# FASCICULE DE BREVET EUROPEEN

(11) **EP 0 699 649 B1**
(45) Date de publication et mention de la délivrance du brevet: **03.11.1999**
(21) Numéro de dépôt: 95202333.1
(22) Date de dépôt: 29.08.1995
(51) Int. Cl.: C07C 17/20, C07C 19/08

(54) **Procédé pour l'hydrofluoration de chloro(fluoro)butane**
Verfahren zur Hydrofluorierung von Chloro(fluoro)butan
Process for the hydrofluorination of chloro(fluoro)butane

(30) Priorité: 05.09.1994 FR 9410684
(43) Date de publication de la demande: 06.03.1996
(73) Titulaire: SOLVAY (Société Anonyme), 1050 Bruxelles (BE)
(72) Inventeur: Pennetreau, Pascal, B-1330 Rixensart (BE); Janssens, Francine, B-1800 Vilvoorde (BE)
(74) Mandataire: Jacques, Philippe

(56) Documents cités:
- EP-A- 0 522 639
- GB-A- 675 372
- US-A- 2 981 762

## Description

La présente invention concerne un procédé pour l'hydrofluoration de chloro(fluoro)butane de formule générale C₄H₅ClₓF₅₋ₓ dans laquelle x = 1 à 5. Elle concerne également l'utilisation de ce procédé pour la préparation du 1,1,1,3,3-pentafluorobutane.

McBee et al. (Ind. Eng. Chem., 39, 1947, p. 420) divulguent un procédé pour la préparation du 1,1,1,3,3-pentafluorobutane, par hydrofluoration du 1,1,1-trichloro-3,3-difluorobutane au moyen de fluorure d'hydrogène en présence d'oxyde mercurique. Ce procédé connu de préparation de 1,1,1,3,3-pentafluorobutane est difficilement exploitable industriellement, d'une part à cause de l'utilisation d'oxyde mercurique et, d'autre part à cause du faible rendement de la réaction d'hydrofluoration (inférieur à 15 %).

La présente invention a pour but de procurer un procédé d'hydrofluoration de chloro(fluoro)butane de formule générale C₄H₅ClₓF₅₋ₓ dans laquelle x = 1 à 5, qui évite les inconvénients sus-mentionnés, dont l'exploitation industrielle est simple, et qui permet en outre d'atteindre un taux de fluoration élevé avec un bon rendement d'hydrofluoration.

A cet effet, l'invention concerne un procédé d'hydrofluoration de chloro(fluoro)butane de formule générale C₄H₅ClₓF₅₋ₓ dans laquelle x = 1 à 5, au moyen de fluorure d'hydrogène et en présence d'un catalyseur choisi parmi les dérivés des métaux des groupes IIIa, IVa, IVb, Va, Vb et VIb du tableau périodique des éléments.

Dans le procédé selon l'invention, on entend désigner par l'expression chloro(fluoro)butane tous les composés, linéaires et ramifiés, de formule générale C₄H₅ClₓF₅₋ₓ dans laquelle x = 1 à 5. L'expression chloro(fluoro)butane incorpore dès lors à la fois les pentachlorobutanes, les chlorofluorobutanes conformes à la formule précédente et les mélanges de deux ou plusieurs de ces composés.

Dans une forme de réalisation particulière du procédé selon l'invention le chloro(fluoro)butane est sélectionné parmi les composés de formule générale CH₃-CClₓF₂₋ₓ-CH₂-CCl_{y}F_{3-y} dans laquelle x = 0, 1 ou 2, y = 0, 1, 2 ou 3 et x + y ≥ 1. Dans cette forme de réalisation, le chloro(fluoro)butane peut notamment comprendre le 1,1,1,3,3-pentachlorobutane CH₃-CCl₂-CH₂-CCl₃ ou les divers chlorofluorobutanes suivants : CH₃-CCl₂-CH₂-CCl₂F, CH₃-CCl₂-CH₂-CClF₂, CH₃-CCl₂-CH₂-CF₃, CH₃-CClF-CH₂-CCl₃, CH₃-CClF-CH₂-CCl₂F, CH₃-CClF-CH₂-CClF₂, CH₃-CClF-CH₂-CF₃, CH₃-CF₂-CH₂-CCl₃, CH₃-CF₂-CH₂-CCl₂F et CH₃-CF₂-CH₂-CClF₂, ainsi que les mélanges de ces composés entre eux.

Selon une première variante de cette forme de réalisation particulière du procédé, le chloro(fluoro)butane est sélectionné parmi le 1,1,3,3-tetrafluoro-1-chlorobutane, le 1,3,3-trifluoro-1,1-dichlorobutane et le 1,1,3-trifluoro-1,3-dichlorobutane.

Selon une autre variante de cette forme de réalisation particulière du procédé selon l'invention, on met en oeuvre du chloro(fluoro)butane obtenu par réaction de chlorure de vinylidène avec du fluorure d'hydrogène, par exemple un sous-produit de la fabrication industrielle du 1-chloro-1,1-difluoroéthane et/ou du 1,1-dichloro-1-fluoroéthane. Dans cette variante de réalisation de l'invention, le chloro(fluoro)butane peut contenir d'autres sous-produits chlorés ou chlorofluorés, tels que du 1,1-dichloro-fluoroéthane, du 1,1,1-trichloroéthane, des hexanes chlorés ou chlorofluorés. Ces impuretés ne gênent pas significativement le déroulement du procédé selon l'invention et peuvent être séparées ultérieurement des produits de réaction obtenus.

Dans le procédé selon l'invention, le catalyseur est choisi parmi les dérivés des métaux des groupes IIIa, IVa, IVb, Va, Vb et VIb du tableau périodique des éléments. On entend par dérivés des métaux des groupes IIIa, IVa, IVb, Va, Vb et VIb du tableau périodique des éléments, les hydroxydes, les oxydes et les sels organiques et inorganiques de ces métaux ainsi que leurs mélanges. De préférence, le catalyseur est choisi parmi les dérivés des métaux des groupes IVa et Va du tableau périodique des éléments, et plus particulièrement parmi les dérivés de l'étain et de l'antimoine. Dans le procédé selon l'invention, les dérivés des métaux du groupe IIIa, IVa, IVb, Va, Vb et VIb du tableau périodique des éléments sont de préférence des sels et ceux-ci sont de préférence choisis parmi les halogénures, et plus particulièrement parmi les chlorures, les fluorures et les chlorofluorures. Des catalyseurs particulièrement préférés selon la présente invention sont les chlorures, les fluorures et les chlorofluorures de l'étain et de l'antimoine, notamment le tétrachlorure d'étain et le pentachlorure d'antimoine. Tout particulièrement préféré est le pentachlorure d'antimoine.

La quantité de catalyseur mise en oeuvre peut varier dans de larges limites. Elle est généralement d'au moins 0,001 mole de catalyseur par mole de chloro(fluoro)butane. De préférence, elle est d'au moins 0,01 mole de catalyseur par mole de chloro(fluoro)butane. Le plus souvent, la quantité de catalyseur mise en oeuvre ne dépasse pas 1 mole de catalyseur par mole de chloro(fluoro)butane. De préférence, elle ne dépasse pas 0,1 mole de catalyseur par mole de chloro(fluoro)butane.

Le rapport molaire entre le fluorure d'hydrogène et le chloro(fluoro)butane mis en oeuvre est généralement d'au moins 4. De préférence, on travaille avec un rapport molaire d'au moins 8. Le rapport molaire entre le fluorure d'hydrogène et le chloro(fluoro)butane mis en oeuvre ne dépasse en générale pas 25. Il est préférable que le rapport molaire ne dépasse pas 18.

La température à laquelle s'effectue l'hydrofluoration est généralement d'au moins 50 °C. De préférence, elle est d'au moins 80 °C. La température ne dépasse en général pas 150 °C. De préférence, elle ne dépasse pas 130 °C. Dans le cas où le catalyseur utilisé est le pentachlorure d'antimoine, les meilleurs résultats sont obtenus à une température d'au moins 100 °C. Dans le cas où le catalyseur utilisé est le tétrachlorure d'étain, les meilleurs résultats sont obtenus à une température d'au moins 120 °C.

Le procédé selon l'invention est de préférence réalisé en phase liquide. Dans ce cas, la pression est choisie de manière à maintenir le milieu réactionnel sous forme liquide. La pression mise en oeuvre varie en fonction de la température du milieu réactionnel. Elle est généralement de 2 bar à 40 bar.

Le procédé selon l'invention peut être réalisé dans tout réacteur construit en un matériau résistant à la température, à la pression et aux réactifs utilisés, notamment au fluorure d'hydrogène.

Le procédé selon l'invention peut être réalisé en discontinu ou en continu. Dans ce dernier cas, il est avantageux de séparer le produit obtenu du milieu réactionnel, et de recycler au réacteur les autres réactifs et produits, notamment la fraction de chloro(fluoro)butane incomplètement fluorée.

Le procédé peut être réalisé dans un système fermé ou ouvert. Dans ce dernier cas, il est avantageux de soutirer du milieu réactionnel, tout ou en partie, le chlorure d'hydrogène formé. Dans une telle technique, on peut par exemple effectuer le procédé dans un réacteur à ébullition surmonté d'une colonne de rectification afin de parfaire la séparation entre le chlorure d'hydrogène et les autres produits.

Le procédé selon l'invention trouve une utilisation avantageuse pour la préparation du 1,1,1,3,3-pentafluorobutane par hydrofluoration de chloro(fluoro)butane de formule générale CH₃-CClₓF₂₋ₓ-CH₂-CCl_{y}F_{3-y} dans laquelle x = 0, 1 ou 2, y = 0, 1, 2 ou 3 et x + y ≥ 1. Dans cette utilisation du procédé selon l'invention, le catalyseur d'hydrofluoration est avantageusement sélectionné parmi les sels inorganiques (de préférence les chlorures, les fluorures et les chlorofluorures) des métaux des groupes IVa et Va du tableau périodique des éléments. La durée de la réaction d'hydrofluoration nécessaire pour assurer un rendement optimal en 1,1,1,3,3-pentafluorobutane est variable en fonction des conditions opératoires et peut être évaluée dans chaque cas particulier par des essais de laboratoire. A la fin de la réaction d'hydrofluoration le 1,1,1,3,3-pentafluorobutane produit peut être récupéré aisément. Un mode opératoire avantageux au laboratoire consiste à refroidir le réacteur à température ambiante et à transvaser son contenu dans une ampoule à décanter remplie d'eau et de tétrachloroéthylène. La phase organique, contenant le 1,1,1,3,3-pentafluorobutane, est ensuite séparée de la phase aqueuse qui contient notamment le fluorure d'hydrogène n'ayant pas réagi. Le 1,1,1,3,3-pentafluorobutane peut ensuite être purifié par distillation.

Les exemples qui suivent sont donnés dans le but d'illustrer l'invention mais ne sont nullement limitatifs.

### Exemple 1

On a utilisé un autoclave de 0,5 l en acier inoxydable HASTELLOY®B2, équipé d'un agitateur, d'une sonde de température, d'un disque de rupture, d'un tube plongeant permettant d'effectuer des prélèvements en phase liquide en cours d'essais et d'une entrée permettant l'introduction des réactifs.

Ce réacteur, préalablement mis sous vide et refroidi à -20 °C, a été chargé avec 109 g d'un mélange contenant 73,2 % en poids de CH₃-CF₂-CH₂-CClF₂ et 19,2 % en poids d'un composé de formule brute C₄H₅Cl₂F₃, le solde (7,6 % en poids) étant constitué de 1,1-dichloro-1-fluoroéthane, de trichloroéthane et d'hexanes chlorofluorés. On y a ajouté successivement 0,036 mole de tétrachlorure d'étain et 7,25 moles de fluorure d'hydrogène.

Le milieu réactionnel a été agité et a ensuite été progressivement chauffé jusqu'à 100 °C et la pression a été réglée à 20 bar. Après 4 heures à 100 °C, la température a été augmentée jusqu'à 120 °C.

L'avancement de la réaction d'hydrofluoration a été suivi par des prélèvements réalisés à intervalles réguliers dans la phase liquide. Ces prélèvements ont été dilués dans du tétrachloroéthylène et leur composition a été déterminée par analyse chromatographique en phase vapeur. Le résultat de ces analyses est repris dans le tableau I.

**TABLEAU I**

| DUREE (1) heures | COMPOSITION % mole/mole C4 (2) | | |
|---|---|---|---|
| | C₄H₅Cl₂F₃ | CH₃-CF₂-CH₂-CClF₂ | CH₃-CF₂-CH₂-CF₃ |
| mélange initial | 21 | 79 | 0 |
| 0 | 11 | 88 | 1 |
| 0,5 | 4 | 93 | 3 |
| 1 | 2 | 94 | 4 |
| 2 | 1 | 94 | 5 |
| 4 | 0,5 | 92 | 7,5 |
| 5,5 | 0 | 85 | 15 |
| 6,5 | 0 | 70 | 30 |
| 7,5 | 0 | 48 | 52 |
| 8,5 | 0 | 37,5 | 62,5 |
| 10,5 | 0 | 21 | 79 |

| | | | |
|---|---|---|---|
| (1) durée à partir du moment où la température dans le réacteur a atteint 100 °C | | | |
| (2) mole C4 = somme des fractions molaires des composés C₄H₅Cl₂F₃, CH₃-CF₂-CH₂-CClF₂ et CH₃-CF₂-CH₂-CF₃. | | | |

Les résultats du tableau I montrent qu'en présence de tétrachlorure d'étain, à 120 °C, la formation du 1,1,1,3,3-pentafluorobutane atteint un rendement proche de 80 % après 6 h de réaction.

### Exemple 2

Le mode opératoire a été analogue à celui de l'exemple 1.

Le réacteur, préalablement mis sous vide et refroidi à -20 °C, a été chargé avec 127 g d'un mélange contenant 2,4 % en poids de CH₃-CF₂-CH₂-CClF₂ et 90,3 % en poids d'un composé de formule brute C₄H₅Cl₂F₃, le solde (7,3 % en poids) comprenant du 1,1-dichloro-1-fluoroéthane, du trichloroéthane et des hexanes chlorofluorés. On y a ajouté successivement 0,042 mole de pentachlorure d'antimoine et 8,45 moles de fluorure d'hydrogène.

Le milieu réactionnel a été agité et a ensuite été progressivement chauffé jusqu'à 100 °C et la pression a été réglée à 20 bar.

L'avancement de la réaction d'hydrofluoration a été suivi par des prélèvements réalisés à intervalles réguliers dans la phase liquide. Ces prélèvements ont été dilués dans du tétrachloroéthylène et leur composition a été déterminée par analyse chromatographique en phase vapeur. Le résultat de ces analyses est repris dans le tableau II.

**TABLEAU II**

| DUREE (1) heures | COMPOSITION % mole/mole C4 (2) | | |
|---|---|---|---|
| | C₄H₅Cl₂F₃ | CH₃-CF₂-CH₂-CClF₂ | CH₃-CF₂-CH₂-CF₃ |
| mélange initial | 97 | 3 | 0 |
| 0 | 15 | 66 | 19 |
| 0,5 | < 0,5 | 62 | 38 |
| 1 | 0 | 52 | 48 |
| 2 | 0 | 43 | 57 |
| 4 | 0 | 34 | 66 |
| 6 | 0 | 31 | 69 |

| | | | |
|---|---|---|---|
| (1) durée à partir du moment où la température dans le réacteur a atteint 100°C | | | |
| (2) mole C4 = somme des fractions molaires des composés C₄H₅Cl₂F₃, CH₃-CF₂-CH₂-CClF₂ et CH₃-CF₂-CH₂-CF₃. | | | |

Les résultats du tableau II montrent qu'en présence de pentachlorure d'antimoine, à 100 °C, la formation du 1,1,1,3,3-pentafluorobutane est rapide, avec un rendement d'environ 70 % après 6 h de réaction.

### Exemple 3

Le mode opératoire mis en oeuvre a été analogue à celui de l'exemple 2. Le réacteur, préalablement mis sous vide et refroidi à -20 °C, a été chargé avec 124 g d'un mélange contenant 2,4 % en poids de CH₃-CF₂-CH₂-CClF₂ et 90,3 % en poids d'un composé de formule brute C₄H₅Cl₂F₃, le solde (7,3 % en poids) comprenant du 1,1-dichloro-1-fluoroéthane, du trichloroéthane et des hexanes chlorofluorés. On y a ajouté successivement 0,041 mole de pentachlorure d'antimoine et 8,2 moles de fluorure d'hydrogène.

Le milieu réactionnel a été agité et a ensuite été progressivement chauffé jusqu'à 120 °C et la pression a été réglée à 20 bar.

Les résultats des analyses chromatographiques sont repris dans le tableau III.

**TABLEAU III**

| DUREE (1) heures | COMPOSITION % mole/mole C4 (2) | | |
|---|---|---|---|
| | C₄H₅Cl₂F₃ | CH₃-CF₂-CH₂-CClF₂ | CH₃-CF₂-CH₂-CF₃ |
| mélange initial | 97 | 3 | 0 |
| 0 | 0 | 25 | 75 |
| 0,5 | 0 | 7 | 93 |
| 1 | 0 | 3 | 97 |
| 2 | 0 | 1 | 99 |
| 4 | 0 | < 0,5 | > 99,5 |

| | | | |
|---|---|---|---|
| (1) durée à partir du moment où la température dans le réacteur a atteint 120 °C | | | |
| (2) mole C4 = somme des fractions molaires des composés C₄H₅Cl₂F₃, CH₃-CF₂-CH₂-CClF₂ et CH₃-CF₂-CH₂-CF₃. | | | |

Les résultats du tableau III montrent qu'en présence de pentachlorure d'antimoine, à 120 °C, la réaction d'hydrofluoration est très rapide, avec un rendement en 1,1,1,3,3-pentafluorobutane supérieur à 90 % après 0,5 h de réaction. La réaction est quasi complète (99 %) après 2 h de réaction.

## Revendications

1. Procédé de fabrication de pentafluorobutane par hydrofluoration de chloro(fluoro)butane de formule générale C₄H₅ClₓF₅₋ₓ dans laquelle x = 1 à 5, au moyen de fluorure d'hydrogène, caractérisé en ce que l'hydrofluoration est effectuée en présence d'un catalyseur choisi parmi les dérivés des métaux des groupes IIIa, IVa, IVb, Va, Vb et VIb du tableau périodique des éléments.

2. Procédé selon la revendication 1, caractérisé en ce que le catalyseur est choisi parmi les dérivés des métaux des groupes IVa et Va du tableau périodique des éléments.

3. Procédé selon la revendication 2, caractérisé en ce que le catalyseur est choisi parmi les dérivés de l'étain et de l'antimoine.

4. Procédé selon l'une quelconque des revendications 1 à 3, caractérisé en ce que le catalyseur est choisi parmi les chlorures, les fluorures et les chlorofluorures.

5. Procédé selon la revendication 4, caractérisé en ce que le catalyseur est le pentachlorure d'antimoine.

6. Procédé selon l'une quelconque des revendications 1 à 5, caractérisé en ce que le catalyseur est mis en oeuvre à raison de 0,001 à 1 mole de catalyseur par mole de chloro(fluoro)butane.

7. Procédé selon l'une quelconque des revendications 1 à 6, caractérisé en ce que le rapport molaire entre le fluorure d'hydrogène et le chloro(fluoro)butane mis en oeuvre est de 4 à 25.

8. Procédé selon l'une quelconque des revendications 1 à 7, caractérisé en ce qu'on réalise l'hydrofluoration à une température de 50° C à 150° C.

9. Procédé selon l'une quelconque des revendications 1 à 8, caractérisé en ce qu'on réalise l' hydrofluoration en phase liquide, à une pression de 2 bar à 40 bar.

10. Procédé selon l'une quelconque des revendications 1 à 9, caractérisé en ce que le chloro(fluoro)butane est sélectionné parmi les composés de formule générale CH₃-CClₓF₂₋ₓ-CH₂-CCl_{y}F_{3-y} dans laquelle x = 0, 1 ou 2, y = 0, 1, 2 ou 3 et x + y ≥ 1.

11. Procédé selon la revendication 10, caractérisé en ce que le chloro(fluoro)butane est sélectionné parmi le 1,1,3,3-tetrafluoro-1-chlorobutane, le 1,3,3-trifluoro-1,1-dichlorobutane et le 1,1,3-trifluoro-1,3-dichlorobutane.

12. Procédé selon la revendication 10 ou 11, caractérisé en ce que le chloro(fluoro)butane est obtenu par la réaction du chlorure de vinylidène avec le fluorure d'hydrogène.

13. Procédé selon la revendication 10, caractérisé en ce que le chloro(fluoro)butane est le 1,1,1,3,3-pentachlorobutane.

14. Utilisation du procédé selon l'une quelconque des revendications 10 à 13 pour la préparation du 1,1,1,3,3-pentafluorobutane.

## Claims

1. Process for the manufacture of pentafluorobutane through hydrofluorination of chloro(fluoro)butane of general formula C₄H₅ClₓF₅₋ₓ in which x = 1 to 5, by means of hydrogen fluoride, characterized in that the hydrofluorination is performed in the presence of a catalyst chosen from the derivatives of metals of groups IIIa, IVa, IVb, Va, Vb and VIb of the Periodic Table of the elements.

2. Process according to Claim 1, characterized in that the catalyst is chosen from the derivatives of metals of groups IVa and Va of the Periodic Table of the elements.

3. Process according to Claim 2, characterized in that the catalyst is chosen from tin and antimony derivatives.

4. Process according to any one of Claims 1 to 3, characterized in that the catalyst is chosen from chlorides, fluorides and chlorofluorides.

5. Process according to Claim 4, characterized in that the catalyst is antimony pentachloride.

6. Process according to any one of Claims 1 to 5, characterized in that the catalyst is used in a proportion of 0.001 to 1 mole of catalyst per mole of chloro(fluoro)butane.

7. Process according to any one of Claims 1 to 6, characterized in that the molar ratio of hydrogen fluoride to the chloro(fluoro)butane used is from 4 to 25.

8. Process according to any one of Claims 1 to 7, characterized in that the hydrofluorination is carried out at a temperature of 50°C to 150°C.

9. Process according to any one of Claims 1 to 8, characterized in that the hydrofluorination is carried out in liquid phase at a pressure of 2 bar to 40 bar.

10. Process according to any one of Claims 1 to 9, characterized in that the chloro(fluoro)butane is selected from the compounds of general formula CH₃-CClₓF₂₋ₓ-CH₂-CCl_{y}F_{3-y} in which x = 0, 1 or 2, y = 0, 1, 2 or 3 and x + y ≥ 1.

11. Process according to Claim 10, characterized in that the chloro(fluoro)butane is selected from 1,1,3,3-tetrafluoro-1-chlorobutane, 1,3,3-trifluoro-1,1-dichlorobutane and 1,1,3-trifluoro-1,3-dichlorobutane.

12. Process according to Claim 10 or 11, characterized in that chloro(fluoro)butane is obtained by the reaction of vinylidene chloride with hydrogen fluoride.

13. Process accordingly to claim 10, characterised in that the chloro(fluoro)butane is 1,1,1,3,3-pentafluorobutane.

14. Use of the process according to any one of Claims 10 to 13 for the preparation of 1,1,1,3,3-pentafluorobutane.

## Patentansprüche

1. Verfahren zur Herstellung von Pentafluorbutan durch Hydrofluorierung von Chlor(fluor)butan der allgemeinen Formel C₄H₅ClₓF₅₋ₓ, in der x = 1 bis 5, mittels Fluorwasserstoff, dadurch gekennzeichnet, daß die Hydrofluorierung in Gegenwart eines Katalysators ausgeführt wird, der unter den Derivaten der Metalle der Gruppen IIIa, IVa, IVb, Va, Vb und VIb des Periodensystems der Elemente ausgewählt ist.

2. Verfahren gemäß Anspruch 1, dadurch gekennzeichnet, daß der Katalysator unter den Derivaten der Metalle der Gruppen IVa und Va des Periodensystems der Elemente ausgewählt ist.

3. Verfahren gemäß Anspruch 2, dadurch gekennzeichnet, daß der Katalysator unter den Derivaten des Zinns und des Antimons ausgewählt ist.

4. Verfahren gemäß einem der Ansprüche 1 bis 3, dadurch gekennzeichnet, daß der Katalysator unter den Chloriden, den Fluoriden und den Chlorfluoriden ausgewählt ist.

5. Verfahren gemäß Anspruch 4, dadurch gekennzeichnet, daß der Katalysator Antimonpentachlorid ist.

6. Verfahren gemäß einem der Ansprüche 1 bis 5, dadurch gekennzeichnet, daß der Katalysator in einer Menge von 0,001 bis 1 Mol Katalysator pro Mol Chlor(fluor)butan eingesetzt wird.

7. Verfahren gemäß einem der Ansprüche 1 bis 6, dadurch gekennzeichnet, daß das eingesetzte Molverhältnis zwischen dem Fluorwasserstoff und dem Chlor(fluor)butan 4 bis 25 beträgt.

8. Verfahren gemäß einem der Ansprüche 1 bis 7, dadurch gekennzeichnet, daß man die Hydrofluorierung bei einer Temperatur von 50 °C bis 150 °C durchführt.

9. Verfahren gemäß einem der Ansprüche 1 bis 8, dadurch gekennzeichnet daß man die Hydrofluorierung in flüssiger Phase bei einem Druck von 2 bar bis 40 bar durchführt.

10. Verfahren gemäß einem der Ansprüche 1 bis 9, dadurch gekennzeichnet, daß das Chlor(fluor)butan unter den Verbindungen der allgemeinen Formel CH₃-CClₓF₂₋ₓ-CH₂-CCl_{y}F_{3-y}, in der x = 0, 1 oder 2, y = 0, 1, 2 oder 3 und x+y ≥ 1, ausgewählt ist.

11. Verfahren gemäß Anspruch 10, dadurch gekennzeichnet, daß das Chlor(fluor)butan unter 1,1,3,3-Tetrafluor-1-chlorbutan, 1,3,3-Trifluor-1,1-dichlorbutan und 1,1,3-Trifluor-1,3-dichlorbutan ausgewählt ist.

12. Verfahren gemäß Anspruch 10 oder 11, dadurch gekennzeichnet, daß das Chlor(fluor)butan durch Reaktion von Vinylidenchlorid mit Fluorwasserstoff erhalten wird.

13. Verfahren gemäß Anspruch 10, dadurch gekennzeichnet, daß das Chlor(fluor)butan 1,1,1,3,3-Pentachlorbutan ist.

14. Verwendung des Verfahrens gemäß einem der Ansprüche 10 bis 13 für die Herstellung von 1,1,1,3,3-Pentafluorbutan.
